# EUROPEAN PATENT SPECIFICATION

(11) **EP 2 271 739 B1**
(45) Date of publication and mention of the grant of the patent: **11.06.2014**
(21) Application number: 09739078.5
(22) Date of filing: 24.04.2009
(51) Int. Cl.: C12N 5/00

(54) **PRODUCT FOR CELL CULTURE**
ZELLKULTURPRODUKT
PRODUIT POUR CULTURE CELLULAIRE

(30) Priority: 28.04.2008 SE 0800963
(43) Date of publication of application: 12.01.2011
(73) Proprietor: GE Healthcare Bio-Sciences AB, 751 84 Uppsala (SE)
(72) Inventor: ALGOTSSON, Mattias, 751 84 Uppsala (SE); BJURLING, Åsa, 751 84 Uppsala (SE)
(74) Representative: Aldenbäck, Ulla Christina
(86) International application number: PCT/SE2009/050430
(87) International publication number: WO 2009/134197

(56) References cited:
- EP-A2- 0 097 907
- US-A1- 2003 138 874
- US-B1- 6 214 618
- BALLEZ J S ET AL: "Plant protein hydrolysates support CHO-320 cells proliferation and recombinant IFN-gamma production in suspension and inside microcarriers in protein-free media.", CYTOTECHNOLOGY MAR 2004 LNKD- PUBMED:19003233, vol. 44, no. 3, March 2004 (2004-03), pages 103-114, XP19236825, ISSN: 0920-9069
- IWATA, MINEO ET AL.: 'Cell-to-substratum adhesion of dissociated embryonic cells of the sea urchin, Pseudocentrotus depressus' WILHELM ROUX,S ARCHIVES OF DEVELOPMENTAL BIOLOGY vol. 193, no. 2, 1984, pages 71 - 77, XP008143451
- JUAN, XU ET AL.: 'Promotion of Differentiation and proliferation of peripheral blood CD34+ cells in vitro by G-CSF' CHINESE MEDICAL JOURNAL vol. 109, no. 5, 1996, pages 372 - 375, PAGE 372, XP008143452
- TURNER, DAVID C. ET AL.: 'Identification of a Cell- Surface Protein Involved in PC12 Cell-Substratum Adhesion and Neurite Outgrowth on Laminin and Collagen' THE JOURNAL OF NEUROSCIENCE vol. 9, no. 9, September 1989, pages 3287 - 3296, XP008143453

## Description

### Field of the invention

The present invention relates to a product for cell culture. More closely, the invention relates to a cell growth surface comprising an animal protein free coating. The coating is derived from a plant protein, preferably soy peptone.

### Background of the invention

Cell culture techniques have become vital to the study of animal cell structure, function and differentiation and for the production of many important biological materials, such as virus vaccines, enzymes, hormones, antibodies, interferon's, nucleic acids and virus vectors for gene therapy. Another important area for cell culture is cell expansion from a small to a large cell population.

For cell culture it is conventional to grow the cells on a cell adhering surface since most mammalian cells and certain other cells are anchorage-dependent to be able to grow. Conventional cell culture in tissue culture treated bottles or other vials give a limited yield of anchorage-dependent cells due to the small surface areas available.

Microcarrier culture introduces new possibilities and for the first time makes possible the practical high yield culture of anchorage-dependent cells. In microcarrier culture cells grow as monolayers on the surface of small spheres which are usually suspended in culture medium by gentle stirring. By using microcarriers in simple suspension culture systems it is possible to achieve yields of several million cells per millilitre and the systems are easily scalable.

In the microcarrier approach, cell culture is realised with beads in a spinner flask or beads packed in columns (perfusion culture). The microcarriers are for example dextran, cellulose or polyethylene based products.

The microcarriers are often provided with an animal protein-derived coating, such as a coating of porcine or bovine gelatin. Leakage of animal protein from conventional microcarrier media is a problem, especially in the production of cells and vaccines for therapy. Thus, it would be desirable to have an animal protein free microcarrier product replacing animal protein containing products, such as porcine collagen-coated microcarriers.

US 6 214 618 describes protein free microcarriers comprising a cross-linked styrene monomer core and divinylbenzene and trimethylamine attached to the surface of the bead. The bead facilitates cell attraction and attachment. This patent also mentions ProNectin F (a genetically engineered protein)-coated polystyrene beads, which uses multiple copies of the cell attachment ligand (RGD) from fibronectin on the surface.

Ballez J.S. et al in Cytotechnology, vol 44, no 3, March 2004, pages 103-114 disclose the growth of an adherent cell line (CHO) on non-woven polyester microcarriers in a medium comprising plant protein hydrolysates. The microcarriers are not modified with plant peptones.

Common nutrients for cell culture include for example different growth factors, hormones, carbohydrates, amino acids, vitamins, lipids and minerals. Traditionally different kinds of animal derived sera were used to help provide these nutrients. Today the cell cultures are often carried out using animal component free media in which different kinds of plant hydrolysates are used to replace the sera [Enhancing performance in cell culture, Babcock et al, Tutorial Bioprocessing, 2007].

### Summary of the invention

The present invention provides a cell growth surface for anchorage-dependent cells wherein the surface has an external layer or coating with combined cell attachment and cell nutrient properties and comprises animal free material.

Thus, the present invention relates to a novel product for cell culture. The product comprises microcarriers provided with a coating derived from plant protein. The preferred plant protein is soy protein, hydrolysed to soy peptone. It was surprising that soy peptone, which is a known cell nutrient, could be attached to a cell culture surface like for example a microcarrier bead and that it had cell attractive properties.

Plant proteins do not have the drawbacks associated with animal proteins. Furthermore, compared to recombinant proteins, plant proteins are easier and cheaper to use in the process of microcarrier production.

In a first aspect, the invention relates to a cell culture product, comprising a cell culture surface provided with a coating derived from an enzymatic hydrolysate of plant protein, i.e. peptones. The surface is a microcarrier.

The microcarrier may be of synthetic origin, such as glass or synthetic polymers like for example polystyrene, or of natural origin, such as dextran, cellulose or agarose or other natural polymers.

Preferably the plant protein is soy protein, pea protein, rice protein or wheat protein. The most preferred peptones are soy peptones that are chemically coupled to the surface. Preferably the plant protein- derived coating or peptones are provided on the surface. The peptones could be attached to the surface by different activation chemistries such as for example epoxy activation using epichlorohydrine (ECH), allylation, NHS-activation and through radical coupling.

If the surface is relatively inert from the start it may have to be activated by for example plasma treatment to introduce some groups like amines and carboxylic acids onto it. These groups could then be used for further activation of the surface using some of the coupling chemistries mentioned earlier and coupling of peptones. It is also possible to first coat the surface with a layer of some polymer, like for example dextran, or epoxysilane that contains groups that enable activation and then couple the peptones onto the polymer layer. The chemistries behind surface activation and coupling are thus well known. A lot of different techniques have been described by for example [Polymer surface modification for the attachment of bioactive compounds, Goodard et al, Progress in Polymer Science, 32 (2007) 698-725].

In a second aspect, the invention relates to use of a cell culture product comprising a cell growth surface provided with a plant protein-derived coating for culturing of adherent cell cultures, wherein the plant protein derived coating comprises peptones and has cell nutrient as well as cell attractive properties. The cell culture product is intended for culture of any anchorage-dependent cells.

### Detailed description of the invention

### Experimental part

The invention will now be more fully described in association with some examples which are not to be construed as limiting for the invention.

Parameters such as cell attachment, cell growth and detachment have been tested.

**Table 1 Materials**

| **Name** | **Article number** | **Supplier** | **Lot/Quality** | **Comment** |
|---|---|---|---|---|
| Soy Peptone E110 | AI885 | Organo Technie | 07206 | Average Mw 1206 daltons |
| Pea Peptone A482 | AI275 | Organo Technie | 06109 | No info of average Mw |
| Epichlorohydrine-activated Sephadex™ gel. | | | Lot: 10016827 1,08% N* | The N content comes from quarternary amines on the qel. |

| | | | | |
|---|---|---|---|---|
| * Values from elemental analysis of dried gels, N% by weight. | | | | |

### Experiment 1: Prototypes using Epichlorohydrine (ECH) -activated gels

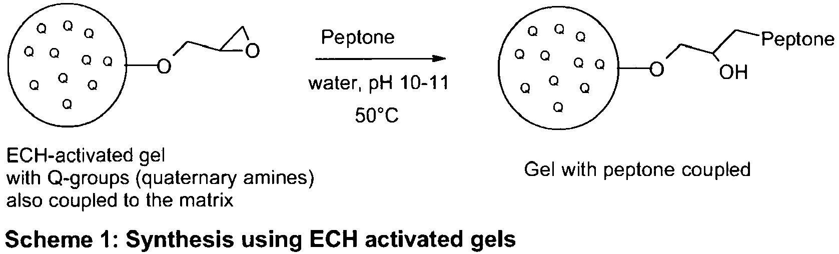

Peptone (4g, see table 2) was dissolved in 10ml of distilled water in a hanging stirrer bar flask. The solution was then warmed to 60°C during stirring. ECH-activated gel was transferred to a glass cylinder and allowed to sediment for approximately one hour, 20ml of the gel was then transferred with a pipette to the flask. Distilled water (5ml) was then added to the flask and the pH adjusted to 10.3-10.4 with sodium hydroxide (50%-solution). After approximately 30 minutes the pH was checked again and adjusted to 10.3-10.4 the temperature was then set to 50°C and the gel left overnight. After around 18 hours the gel was transferred to a glass filter and washed (see table 2). The gel was then dried according to the general procedure. The nitrogen content was then measured by elemental analysis.

**Table 2: Couplings with ECH-activated gels**

| **Name** | **Peptone** | **ECH-activated gel Lot nr** | **Washing*** |
|---|---|---|---|
| U1692057A | Soy Peptone E110 | 10016827 | 1. Warm (50°C) 0.9% NaCl (1GV x3) |
| | | | 2. TRIS and then NaOAC, (x3) |
| | | | 3. 0.9% NaCl (x3) |
| U1692059A | Pea Peptone A482 | 10016827 | 1. Warm (50°C) 0.9% NaCl (1GV x3) |
| | | | 2. TRIS and then NaOAC, (x2) |
| | | | 3. 0.9% NaCl (x3) |

| | | | |
|---|---|---|---|
| * TRIS-buffert: 0.1M TRIS, 0.5M NaCl, pH 8 NaOAc-buffert: 0.1M NaOAc, 0.5M NaCl, pH 4 GV = Gel Volume | | | |

### General procedure for drying of gels

The gel was transferred to a beaker and allowed to sediment for at least 30 minutes. The supernatant was then removed and acetone (approximately 1 gel volume) was added. The slurry was then thoroughly mixed and left for at least 1 h. This procedure was then repeated with a new gel volume of acetone and the gel was this time left for at least 30 minutes. This procedure was then repeated 2-3 times until the gel was completely shrunken into a white powder. The gel was finally washed on a glass filter with acetone and then dried in an oven (70°C) over night.

### Experiment 2: Screening experiments with Vero cells in microtiter plates

The microcarriers were washed twice in PBS and autoclaved. Before inoculation the carriers were washed with cultivation medium containing Penicillin/Streptomycin. The cultivation medium was DMEM/Ham's F12 1:1 (Biochrom, Berlin, Germany) with 4 mM L-glutamine, 0.1 % soy peptone (Hy Pep 1510, Quest, Naarden, the Netherlands) and 0.01 % β-Cyclodextrin (Roquette, France). For the prototypes containing plant hydrolysates a swelling volume of 14 ml/g was assumed. Suspensions with a carrier concentration of 6 g/l were prepared in cultivation medium. Experiments were done in duplicates on 12 well plates with a working volume of 2 ml/well. 1 ml of each carrier suspension was added to the respective wells and incubated over night in an atmosphere containing 7% CO2. The following day 1 ml inoculum containing 2 E+05 cells/ml was added. The final carrier concentration was therefore 3 g/l, the starting cell concentration 1 E+05 cells/ml
To assess cell morphology microphotographs were taken 6, 24, 48, and 96 hours after inoculation (see table 4).

### Experiment 3: Screening experiments with Vero cells and hMSC cells in microtiter plates

### Preparation of micro carriers for Vero cells:

Approximately 1 ml settled gel from each sample was transferred to a 15 ml tube and 5 ml PBS was added and well mixed. This wash step was repeated four times. Between each wash the carriers were settled.
After that the micro carriers were autoclaved (20 minutes, 121°C).
Preparations of micro carriers were performed under sterile conditions after the sterilization.
Before adding the cells, the micro carriers were equilibrated twice in basal medium with the same procedure as the washing step. After media removal from the last equilibrating step, 4 ml complete medium were added and the carriers were stored at +2 - 8°C.

### Start of the experiment with Vero cells:

The supernatant from the samples were removed and an equal volume of complete medium was added to get a 50% bead solution.
The experiment with Vero cells consist of two samples, two positive controls and one negative control. Four wells for each sample, totally 20 wells at two plates.

Each day cells from one well were stained with hematoxylin. The cells were transferred to a tube and washed once with 1 ml PBS and 0.2 ml hematoxylin were added and incubated for 1 hour at room temperature.

800 µl medium and 40 µl of the bead solution was added/well. The plates were equilibrated at 37°C, 5% CO₂ for at least 1 hour. After that the cells were added. Cells were incubated with the beads for 3 hours at 37°C and 5% CO₂ and then the beads were transferred to new wells. Cell attachment and spreading on the beads were studied in the microscope at 4, 24, 46 and 69 hours. Notes and photos were taken. Results are shown in Table 5.

After 96 hours a detachment experiment was done. The beads were transferred to a tube, washed once with PBS and then 0.5 ml Trypsin/EDTA was added. The beads were transferred to a micro titer plate and inspected by microscope to see if the cells detached. Results are shown in table 7.

### Preparation of micro carriers for hMSC:

In this experiment two of the prepared prototypes that showed comparable results to Cytodex from the experiment with Vero cells were used (together with the same controls and also adding Cytodex 2).

### Start of the experiment with hMSC:

The supernatant from the samples were removed and an equal volume of complete medium was added to get a 50% bead solution and then the experiment was performed in the same manner as for Vero cells.

The experiment with hMSC consisted of two samples, three positive controls and one negative control, a total of 40 wells in four plates.

The cells were stained with hematoxylin according to the procedure described above. The evaluation was done after 4, 24, 48 and 72 hours. Notes and photos were taken. Results are shown in Table 6.
After 120 hours a detachment experiment was performed. The beads were transferred to a tube, washed once with PBS and then 0.5 ml Trypsin/EDTA was added. The beads were transferred to a microtiter plate and inspected by microscope to see if the cells detached. Results are shown in table 8.

### RESULTS

**Table 3 Synthesis**

| Prototype | Ligand | Starting gel | N% (Elemental analysis) | N% from ligand |
|---|---|---|---|---|
| U1692057A | Soy Peptone E110 | 10016827 (1.08% N) | 1,33 | 0,25 |
| U1692059A | Pea Peptone A482 | 10016827 (1.08% N) | 1,24 | 0,16 |

### Screening experiments with Vero cells in microtiter plates (experiment 2)

### Criteria for the arbitrary performance units

Attachment but no cell growth: 10
Attachment and some spreading after 6h but no cell growth: 10
Attachment but no spreading after 6h, cell growth (at least some of the carriers confluent) after 96h: 30
Attachment and spreading after 6h, cell growth (at least some of the carriers confluent) after 96h: 70

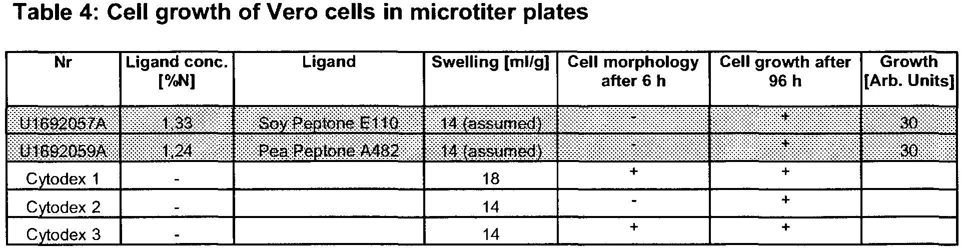

### Screening experiments with Vero and hMSC in microtiter plates

### (experiment 3)

Note: + signs in the tables indicate good cell growth.

**Table 5 Experiment with Vero cells on micro carriers**

| **Plate 1** | | | | | | | | | | | |
|---|---|---|---|---|---|---|---|---|---|---|---|
| **Well** | | **Ligand** | **Ligand-content (mmol/g)** | **Cells binding to beads 3h** | **Cells spreading on beads** | | | | **Note** | | |
| | | | | | **3h** | **24h** | **48h** | **72h** | **24h** | **48h** | **72h** |
| 1 | 10007610 | - | - | - | - | - | - | - | Negative control | - | - |
| 2 | Cytodex 3 | - | - | + | + | +++ | (++++) | ++++ | - | - | - |
| | | | | | | | | | | | |

| **Plate 2** | | | | | | | | | | | |
|---|---|---|---|---|---|---|---|---|---|---|---|
| **Well** | | **Ligand** | **Ligand-content (% N)** | **Cells binding to beads** | **Cells spreading on beads** | | | | **Note** | | |
| | | | | **3h** | **3h** | **24h** | **48h** | **72h** | **24h** | **48h** | **72h** |
| 1 | Cytodex 1 | - | - | + | + | (+++) | (++++ ) | ++++ | - | - | - |
| 2 | U1692057A | Soy Peptone E 110 | 1,33 | + | + | +++ | (++++ ) | ++++ | Comparable with the control | Comparable with the control | Comparable with the control |
| 3 | U1692059A | Pea Peptone A 482 | 1,24 | + | + | +++ | (++++ ) | ++++ | Comparable with the control | Comparable with the control | Comparable with the control but more uneven |

**Table 6 Experiment with hMSC on micro carriers**

| **Plate 1** | | | | | | | | | | | | |
|---|---|---|---|---|---|---|---|---|---|---|---|---|
| **Well** | | **Ligand** | **Ligand-content (mmol/g)** | **Cells binding to beads** | **Cells spreading on beads** | | | | **Note** | | | |
| | | | | **3h** | **3h** | **24h** | **48h** | **72h** | **24h** | | **48h** | **72h** |
| 1 | 10007610 | - | - | - | - | - | - | - | Negative control | | - | - |
| 2 | Cytodex 1 | - | - | + | + | ++ | (+++) | +++ | - | | - | - |
| | | | | | | | | | | | | |

| **Plate 2** | | | | | | | | | | | | |
|---|---|---|---|---|---|---|---|---|---|---|---|---|
| **Well** | | **Ligand** | **Ligand-content (% N)** | **Cells binding to beads** | **Cells spreading on beads** | | | | **Note** | | | |
| | | | | **3h** | **3h** | **24h** | **48h** | **72h** | **24h** | **48h** | **72h** | |
| 1 | Cytodex 2 | - | - | + | + | + | ++ | ++ | - | - | - | |
| 2 | U1692057A | Soy Peptone E 110 | 1,33 | + | + | ++ | (+++) | +++ | - | - | Better than Cytodex 2 | |
| 3 | U1692059A | Pea Peptone A 482 | 1,24 | + | + | ++ | (+++) | +++ | - | - | Better than Cytodex 2 | |

| **Plate 3** | | | | | | | | | | | | |
|---|---|---|---|---|---|---|---|---|---|---|---|---|
| **Well** | | **Ligand** | **Ligand-content (mmol/g)** | **Cells binding to beads** | **Cells spreading on beads** | | | | **Note** | | | |
| | | | | **3h** | **3h** | **24h** | **48h** | **72h** | **24h** | | **48h** | **72h** |
| 1 | Cytodex 3 | - | - | + | + | ++ | (+++) | +++ | - | | - | - |

**Table 7 Detachment experiment with Vero cells**

| **Prototype** | **Ligand** | **Ligand-content (%N)** | **Growth of Vero cells after 96h** | **Detachment of Vero cells after 96h > 98%** |
|---|---|---|---|---|
| Cytodex 1 | - | - | ++++ | 15 min |
| Cytodex 3 | - | - | ++++ | 20 min |
| U1692057A | Soy Peptone E 110 | 1,33 | ++++ | 20 min |
| U1692059A | Pea Peptone A 482 | 1,24 | ++++ | 20 min |

**Table 8 Detachment experiment with hMSC cells**

| **Prototype** | **Ligand** | **Ligand-content (%N)** | **Growth of hMSC after 72h** | **Detachment of hMSC after 120h < 80%** |
|---|---|---|---|---|
| Cytodex 1 | - | - | +++ | >35 min |
| Cytodex 2 | - | - | ++ | >35 min |
| Cytodex 3 | - | - | +++ | >35 min |
| U1692057A | Soy Peptone E 110 | 1,33 | +++ | >35 min |
| U1692059A | Pea Peptone A 482 | 1,24 | +++ | >35 min |

## Claims

1. A cell culture product, comprising a cell growth surface provided with a coating comprising an enzymatic hydrolysate of plant protein, i.e. peptones, wherein said cell growth surface is a microcarrier.

2. Cell culture product according to claim 1, wherein the product is of natural or synthetic origin.

3. Cell culture product according to one or more of the above claims, wherein the peptones are derived from soy, wheat, rice or pea protein.

4. Cell culture product according to one or more of the above claims, wherein the peptones are chemically coupled to the cell growth surface.

5. Use of a cell culture product according to any of the above claims for culturing of adherent cell cultures.

6. Use of a cell culture product according to claim 5 for the growth of mammalian cell cultures.

7. Method for preparing a cell culture product, comprising providing a cell growth surface with a coating comprising an enzymatic hydrolysate of plant protein, i.e. peptones, wherein the cell growth surface is a microcarrier.

## Patentansprüche

1. Zellkulturprodukt, umfassend eine Zellwachstumsoberfläche, versehen mit einer Beschichtung, umfassend ein enzymatisches Hydrolysat von Pflanzenprotein, d.h. Peptonen, wobei die Zellwachstumsoberfläche ein Mikroträger ist.

2. Zellkulturprodukt nach Anspruch 1, wobei das Produkt natürlichen oder synthetischen Ursprungs ist.

3. Zellkulturprodukt nach einem oder mehreren der vorangehenden Ansprüche, wobei die Peptone von Soja-, Weizen-, Reis- oder Erbsenprotein abgeleitet sind.

4. Zellkulturprodukt nach einem oder mehreren der vorangehenden, wobei die Peptone chemisch an die Zellwachstumsoberfläche gekoppelt sind.

5. Verwendung eines Zellkulturprodukts nach einem der vorangehenden Ansprüche zum Kultivieren anhaftender Zellkulturen.

6. Verwendung eines Zellkulturprodukts nach Anspruch 5 für das Wachstum von Säugetierzellkulturen.

7. Verfahren zum Herstellen eines Zellkulturprodukts, umfassend das Versehen einer Zellwachstumsoberfläche mit einer Beschichtung, umfassend ein enzymatisches Hydrolysat von Pflanzenprotein, d.h. Peptonen, wobei die Zellwachstumsoberfläche ein Mikroträger ist.

## Revendications

1. Produit de culture cellulaire, comprenant une surface de croissance cellulaire pourvue d'un revêtement comprenant un hydrolysat enzymatique de protéine végétale, c'est-à-dire des peptones, dans lequel ladite surface de croissance cellulaire est une microporteuse.

2. Produit de culture cellulaire selon la revendication 1, dans lequel le produit est d'origine naturelle ou synthétique.

3. Produit de culture cellulaire selon une ou plusieurs des revendications précédentes, dans lequel les peptones sont tirées de protéines de soja, de froment, de riz ou de pois.

4. Produit de culture cellulaire selon une ou plusieurs des revendications précédentes, dans lequel les peptones sont couplées chimiquement à la surface de croissance cellulaire.

5. Utilisation d'un produit de culture cellulaire selon l'une quelconque des revendications précédentes pour cultiver des cultures de cellules adhérentes.

6. Utilisation d'un produit de culture cellulaire selon la revendication 5 pour la croissance de cultures de cellules mammifères.

7. Procédé de préparation d'un produit de culture cellulaire, comprenant l'étape consistant à pourvoir une surface de croissance cellulaire d'un revêtement comprenant un hydrolysat enzymatique de protéine végétale, c'est-à-dire des peptones, dans lequel la surface de croissance cellulaire est une microporteuse.
